# EUROPEAN PATENT APPLICATION

(11) **EP 1 591 536 A1**
(43) Date of publication of application: **02.11.2005**
(21) Application number: 04291102.4
(22) Date of filing: 29.04.2004
(51) Int. Cl.: C12Q 1/68

(54) **Method of selecting sunflower genotypes with high oleic acid content in seed oil**

(71) Applicant: INSTITUT NATIONAL DE LA RECHERCHE AGRONOMIQUE, 75338 Paris Cedex 07 (FR); Monsanto SAS, 69500 Bron (FR)
(72) Inventor: Berville, André, 34000 Montpellier (FR); Lacombe, Séverine, 12450 Luc (FR); Veillet, Stanislas, 64600 Anglet (FR); Granier, Christel, 64100 Bayonne (FR); Léger, Sandrine, 64600 Anglet (FR); Jouve, Philippe, 82700 Montech (FR)
(74) Representative: Gillard, Marie-Louise

(57) **Abstract**

The present invention relates to the selection of sunflower genotypes with high oleic acid content in seed oil. The invention concerns more particularly molecular markers useful for a rapid and easy selection of sunflower lines and then sunflower hybrids capable of producing seeds having high oleic acid content.

## Description

The present invention relates to the selection of sunflower genotypes with high oleic acid content in seed oil. The invention concerns more particularly molecular markers useful for a rapid and easy selection of sunflower lines and then sunflower hybrids capable of producing seeds having high oleic acid content.

In the present description, the expression "high oleic acid content seeds" designates seeds containing more than 60% of oleic acid.

### BACKGROUND OF THE INVENTION

Since the 1960s, vegetable oils rich in unsaturated fatty acids have become particularly important due to the relationship established between the saturated fatty acids present notably in animal fats and the cholesterol level increase.

Most of the fatty acids in vegetable oils are fatty acids having 16 or 18 carbon atoms. The C16-fatty are generally saturated fatty acids (16:0 = palmitic acid). Conversely, the C18 fatty acids are either saturated (18:0 = stearic acid) or unsaturated having 1, 2 or 3 double bonds (18:1 = oleic acid; 18:2 = linoleic acid; 18:3 = linolenic acid).

Certain unsaturated fatty acids, notably linolenic acid, are not synthesized by the human body and are of an insufficient amount in fats of animal origin. Among the unsaturated fatty acids, the mono-unsaturated 18:1 oleic acid, has properties which is particularly suitable for preventing cardiovascular diseases. Moreover, High oleic acid content oils are more resistant to heating and are thus recommended for frying.

The fatty acids composition in vegetable oils deriving from seeds is variable depending on the oleaginous plant. Olive oil has naturally high 18:1 content (around 70%), nevertheless, its non-lipid part of the oil decreases the interest of this oil. Normal sunflower (hereinafter named LO varieties) oil contains mainly linoleic acid and high levels of phytosterols and tocopherol conferring hypocholesterolemic and anti-oxidant properties to the non-lipid part of the oil. Sunflower lines and hybrids, which have high 18:1 content in their seeds (hereinafter named HO varieties) have been obtained by selection programs from the HO Pervenets mutant, said mutant being obtained by chemical mutagenesis (1). They are particularly interesting since they have the combined benefits of oleic acid and the non-lipid part of the sunflower oil (phytosterol and tocopherol). They therefore respond to the requirement of quality by consumers.

In addition to their dietary benefits, vegetable oils are of significant industrial interest. Indeed, they represent a source of fatty acids as starting material used in the lipochemical industry (detergents, paints, cosmetics). Vegetable oil from the HO sunflower type is a substantially pure industrial source of 18:1, that reduces the purification steps.

In order to respond to the needs of vegetable oils of food or industrial interest, the improvement of the oleaginous varieties is concerned with the control and the change of their fatty acid compositions by means of conventional selection programs, but also by mutagenesis and transgenesis.

The chemical mutagenesis carried out by Soldatov in 1976 (*1*) on a sunflower population allowed the Pervenets mutant population to be obtained. The average content of 18:1 of the seeds from this population is higher than 65%, the individual contents being between 60 and 80% whereas in normal LO varieties this content is about 20%. The Pervenets population was distributed throughout the world and used in many selection programs in order to convert selected genotypes with low 18:1 content into genotypes having 18:1 content in their seeds higher than 80%.

During the conversion process of the normal LO material into HO one, the selection is generally based on the 18:1 content of the seeds. This phenotypic determination does not allow a rapid and early detection of HO genotypes and cannot distinguish between homozygote and heterozygote genotypes for the mutation. It is therefore necessary to have selection markers at genomic level allowing earlier and rapid determination of the HO phenotype homozygous for the mutation during conversion process.

The accumulation of the 18:1 in the seeds is mainly dependent on two enzymatic reactions: the upstream desaturation of 18:0 into 18:1 and the downstream desaturation of 18:1 into 18:2. Studies carried out by Garcés and Mancha in 1989 and 1991 (*2, 3*) demonstrated that the HO phenotype is associated with a marked activity decrease of the Δ12-desaturase enzyme, which catalyses the desaturation of 18:1 into 18:2 in the HO seeds during the critical stages of constructing the lipidic stocks, explaining the 18:1 accumulation. Kabbaj et al (4) subsequently demonstrated that one HO genotype presents a significant decrease in the Δ12-desaturase mRNA levels in the seeds during the critical stages of producing the lipidic stocks compared to 2 LO genotypes. This decrease explains the decrease of the amount of enzyme and therefore of Δ12-desaturase activity already demonstrated. Hongtrakul et al. (*5*) and then Lacombe et al. (*6, 7*) showed that HO lines derived from Pervenets mutant carry specific restriction fragment length polymorphisms (RFLPs) revealed using a Δ12-desaturase cDNA as a probe. These RFLPs determine the Δ12 HO specific allele, Δ12HOS. The normal LO lines do not carry the Δ12HOS allele but another allele named Δ12LOR at this locus (named Δ12HL locus) (*6, 7*). Studying the inheritance of the phenotype HO in an F2 population, Lacombe et al. (*6*) revealed that the HO phenotype co-segregated with the Δ12HOS allele pointing out that the Pervenets mutation is carried or genetically tightly linked to the Δ12HOS allele. In another segregating population (recombinant inbred lines F6 population), Lacombe et al. (*7*) showed that all the HO lines carry the Δ12HOS allele. However, the recombinant lines carrying Δ12HOS are equally shared into HO or LO classes. The absence of HO plants carrying the Δ12LOR allele eliminates the occurrence of a recombination event between the Δ12HL locus and the locus containing the Pervenets mutation. In the genetic background of this population, the HO phenotype is therefore due to two independent loci: the Δ12HL locus carrying the Δ12HOS allele and consequently the Pervenets mutation, and another locus, where an allele suppresses the effect of the Δ12HOS allele leading to restore the LO phenotype.

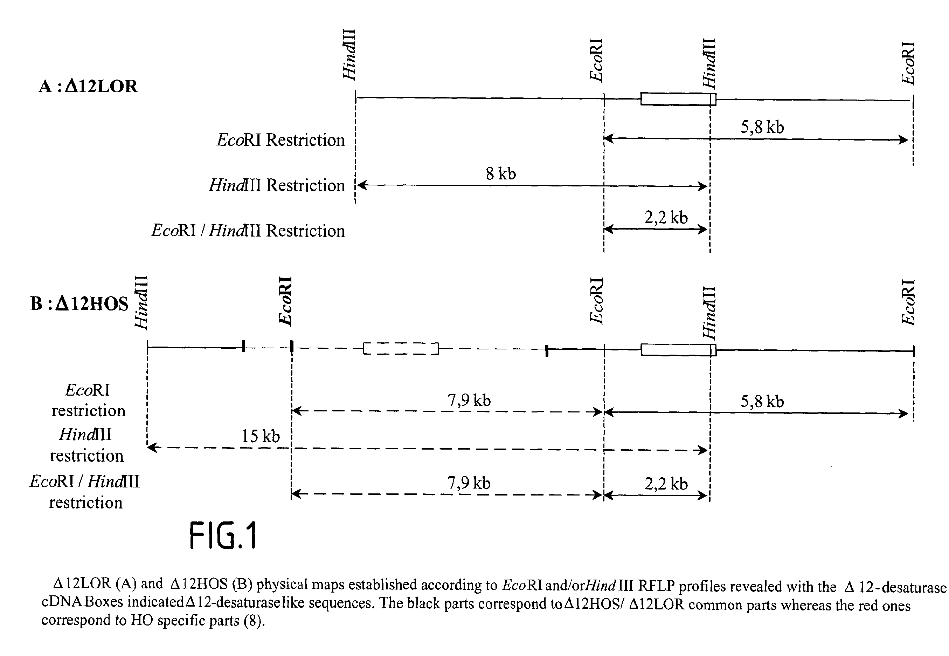

Once the coincidence or the very tightly genetic linkage between the Pervenets mutation and the Δ12HOS allele revealed, Lacombe et al. (*8*) established physical maps of the Δ12HOS and Δ12LOR alleles at the Δ12HL locus (Fig 1). For this purpose, they used the HO and LO RFLP profiles revealed with the Δ12-desaturase cDNA used as a probe. The Δ12LOR allele in LO genotypes corresponds to a 5.8 kb *EcoRI* and to a 8 kb *Hind*III fragments carrying Δ12-desaturase like sequences. The double digest with *Eco*RI + *Hind*III leads to a 2.2 kb fragment. For the Δ12HOS allele in HO genotypes, the 5.8 kb *Eco*RI fragment is still present but another 7.9 kb *Eco*RI extra fragment is also revealed. With *Hind*III*,* the 8 kb fragment revealed in LO genotypes lengthens to 16 kb in HO genotypes. With the *Eco*RI + *Hind*III double digest, the Δ12HOS allele displayed the 2.2 kb fragment revealed in Δ12LOR allele plus the 7.9 kb *Eco*RI extra fragment. According to all these data, the Δ12HOS and Δ12LOR allele physical maps were established. The Δ12LOR allele carries one region whereas the Δ12HOS allele carries 2 adjacent regions with Δ12-desaturase like sequences: the Δ12HOS / Δ12LOR common region determined by the common 5.8 kb *Eco*RI fragment and a HO specific region determined by the 7.9 kb *Eco*RI extra fragment (Fig 1, ref *8*).

Studying genomic clones carrying Δ12-desaturase like sequence from a HO genotype genomic library, Lacombe et al. (*8*) revealed that the Δ12HOS / Δ12LOR common fragment should carry a functional Δ12-desaturase gene interrupted by a 1686bp intron in the 5'UTR part of the gene.

### SUMMARY OF THE INVENTION

The present invention relates to molecular markers that are strictly linked to genetic factors involved in 18:1 accumulation in seed oil of HO genotypes of sunflower: the Pervenets mutation and another independent factor, the supole factor.

The invention relates to PCR molecular markers that partly amplify the Pervenets mutation. It also relates to a SSR marker (15-17 TTA motifs) in the intron of the Δ12-desaturase functional gene adjacent to the mutation that enables to map the gene locus in most of segregating populations and to select genotypes carrying the Pervenets mutation.

The invention also relates to specific markers of a genetic factor suppressing the Pervenets mutation effect. The presence of the suppressor (supole) is revealed in genotypes by combining the presence of the Δ12HOS allele and the low 18:1 content in seed oil.

All these molecular markers may represent advantageous and useful tools in selection programs for rapid screening and early detection of HO genotypes producing seeds with a high 18:1 content, carrying the Pervenets mutation without the unfavourable supole factor by means of the PCR technology.

The invention also relates to processes for the selection of sunflower producing seeds with a high oleic acid content.

The present invention also relates to primers useful for nucleic acid amplification and to combinations thereof.

Finally, the invention concerns the test kits for selecting sunflower having seeds with a high content of oleic acid which contain at least one of the above combinations.

### DETAILED DESCRIPTION OF THE INVENTION

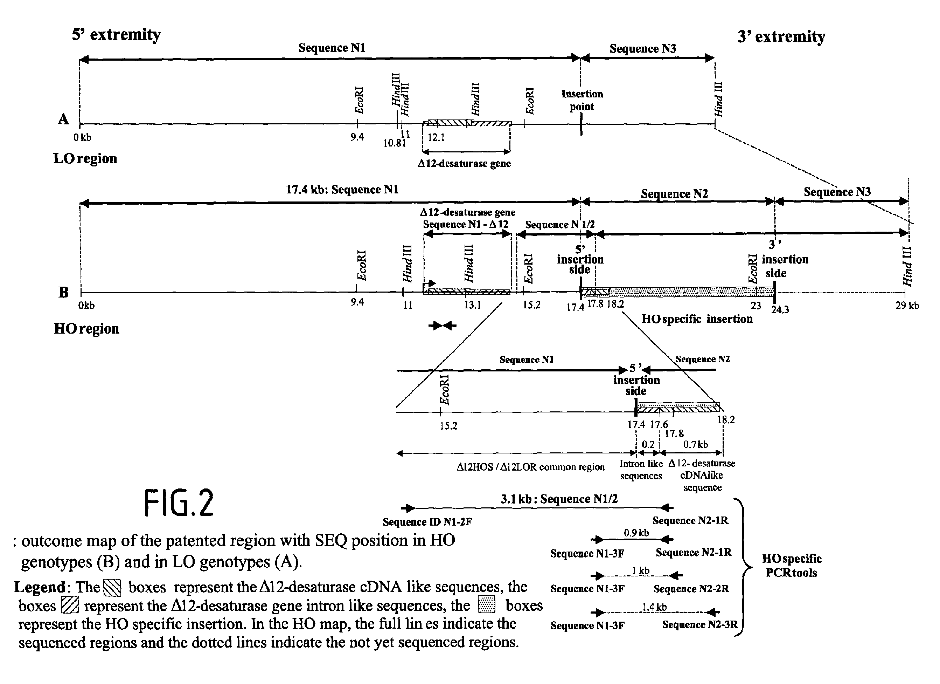

The present invention relates to Pervenets mutation specific molecular markers located in a 29 kb region, a map thereof is represented on Fig 2. This region carrying these molecular markers is divided in 3 sequences, from 5' to 3':
- sequence N1 having 6.662 kb: Δ12HOS / Δ12LOR common part carrying a Δ12-desaturase gene (SEQ ID NO:1) ;
- sequence N2: HO specific insertion; (this sequence has been partially sequenced (SEQ ID NO:2) ;
- sequence N3: the 3' adjacent part of the HO specific insertion fragment.

The molecular marker of the invention comprises the isolated nucleic acid SEQ ID N° 3 having 872 bp or a sequence having a high degree of homology with sequence SEQ ID N°3. Said nucleic acid sequence is a part of sequence SEQ ID NO:4 having 3026 bp and being identified as sequence N1/2 on Fig 2. The marker having SEQ ID NO:3 is located between 2154 bp and 3026 bp of SEQ ID NO:4.

In the present description, a high degree of homology denotes a homology (ratio of the identical nucleotides to the total number of nucleotides) of at least 85 %, and preferably 90 %, for the nucleotide sequences when they are aligned according to the maximum homology by the optimal sequence alignment method of ALIGN. This method is used especially in the GCG software of Devereux et al. (9).

The invention also relates to the isolated nucleic acid fragments having 10-30 bp and which hybridize with SEQ N2 (SEQ ID NO:2). Said fragments are particularly useful tools as primers for nucleic acid amplification, for example PCR amplification.

Examples of said fragments are the fragments having the following sequences:

sequence N2-1R: 5′-AGCGGTTATGGTGAGGTCAG-3′ (SEQ ID N°7)

sequence N2-2R: 5'-ACAAAGCCCACAGTGTCGTC-3' (SEQ ID N°8)

sequence N2-3R: 5′-GCCATAGCAACACGATAAAG-3′ (SEQ ID N°9)

The invention also relates to the primer pairs useful for nucleic acid amplification, which comprise:
1) as the first member of the pair a nucleic acid fragment having 1 to 30 bp and which hybridises with sequence N1 (SEQ ID N°1) and
2) as the second member of the pair a nucleic acid fragment having 10 to 30 bp and which hybridises with SEQ ID NO:2.

Examples of such pairs of primers comprise as:
1) first member : sequence N1-2F :     5'-CAAACCACCACCCACTAAC-3' (SEQ ID N°5) or sequence N1-3F :	5′-GAGAAGAGGGAGGTGTGAAG-3′ (SEQ ID N°6)
2) second member :

sequence N2-1R: 5'-AGCGGTTATGGTGAGGTCAG-3' (SEQ ID N°7)

sequence N2-2R: 5'-ACAAAGCCCACAGTGTCGTC-3' (SEQ ID N°8)

sequence N2-3R:      5'-GCCATAGCAACACGATAAAG-3' (SEQ ID N°9)

In the present description, acid nucleic sequences codes end by a number whereas acid nucleic fragments useful as primers end by F or R depending on they are designed forward or reverse, respectively. These references are reported on the map of the 29 kb region (Fig 2).

The invention also relates to the isolated SSR nucleic acid sequence SEQ ID N° 10 having 45 bp with 16 TTA repeat microsallelites TTA. This sequence involving a polymorphism associated with the Pervenets mutation may be used in selection programs for rapidly identifying the locus of the Pervenets mutation.

Said SSR sequence is located in the intron of the oleate-desaturase gene besides the Pervenets insertion.

Therefore, said SSR sequence is very useful for mapping the oleate-desaturase gene in the genome of sunflower plants to be selected.

The invention also concerns the molecular marker specific for mapping the oleate-desaturase gene in the genome of the sunflower to be selected which comprises the nucleic acid sequence SEQ ID N° 10 or a sequence having a high degree of homology with sequence SEQ ID N° 10.

The invention also concerns the isolated nucleic acid fragments SEQ ID N° 11 and SEQ ID N° 12. Said fragments are useful as primers for amplifying the above SSR sequence:

sequence N1-1F : 5' -TTGGAGTTCGGTTTATTTAT- 3' (SEQ ID N° 11)

sequence N1-1R : 5' -TTAGTAAACGAGCCTGAAC- 3' (SEQ ID N° 12)

All the isolated nucleic acid sequences and fragments of the invention may be obtained by chemical synthesis following the conventional methods well known by the person skilled in the art.

The invention also relates to a process for selecting sunflower having seeds with a high content of oleic acid which comprises the steps of:
- extracting the genomic DNA;
- amplifying said genomic DNA by means of primer pair, said pair being constituted with a first member of nucleic acid fragment having 10 to 30 bp, which hybridises with sequence N1 (SEQ ID NO:1) and a second member of nucleic acid fragment having 10 to 30 bp, which hybridises with sequence N2 (SEQ ID NO:2);
- hybridizing the amplified DNA fragment with the labelled sequence SEQ ID N° 3 and
- isolating the genotypes giving a positive hybridisation signal.

This process allows the selection of genotypes having the Pervenets mutation without needing to determine the oleic acid content of the seeds, which considerably reduces the selection time.

Following an advantageous embodiment of this process, the Pervenets mutation is firstly mapped in most of progenies (most of sunflower line couples display polymorphism for the SSR) by a process comprising the steps of:
- extracting the genomic DNA;
- amplifying said genomic DNA by means of primers SEQ ID N° 11 and SEQ ID N° 12;
- isolating the clones having a DNA fragment length which is different from a reference clone without the Pervenets mutation.

The thus selected clones have the Pervenets mutation and are used as such in the above invention process.

Examples of the primer pairs useful in the invention process are the:
- isolated nucleic acid fragments SEQ ID NO:5 and SEQ ID NO:6 as first member of said pair, and
- isolated nucleic acid fragments SEQ ID NO:7 to SEQ ID NO:9 as second member of said pair.

The invention also relates to the molecular marker constituted by the isolated nucleic sequence SEQ ID NO:13 having 763 bp, which is common to LO and HO regions and located over the insertion point of the Pervenets mutation. The insertion point is located between the bp 83 and bp 366 of SEQ ID NO:13.

The invention also relates to the isolated nucleic acid fragments having 10-30 bp and which hybridise with SEQ ID NO:13. Said fragments are also useful tools as primers for nucleic acid amplification, for example for PCR amplification.

Examples of said fragments are the fragments having the following sequences:

sequence N2-1F : 5'-TTTTACTCTTTGTTATAATAG-3' (SEQ ID N°14)

sequence N2-2F : 5'-ACACTAACACTCATTACATTCG-3' (SEQ ID N°16)

sequence N2-3F : 5'-AAAGCAAAAACACCGTGATTC-3' (SEQ ID N°18)

sequence N3-1R : 5'-TTTTTAGTTCATGGAATCAC- 3' (SEQ ID N°15)

sequence N3-2R : 5'-CCTAAAGCTCTGTAGATTTT- 3' (SEQ ID N°17)

sequence N3-3R : 5'-GGTGTTATTATTCAGCCTGAA-3' (SEQ ID N°19)

The invention also relates to the process for amplifying the region across the insertion Pervenets mutation site both in LO and HO genotypes. Said process comprises the steps of :
- extracting the genomic DNA;
- amplifying said genomic DNA by means of primer pair, said pair being constituted with a first member of nucleic acid fragments having 10 to 30 bp which hybridises with sequence N1 (SEQ ID N0:13).

In all the invention processes, the amplification step may be carried out by any nucleic amplification method known by the person skilled in the art, for example by the well-known PCR amplification method.

A 6662 kb region carrying the Δ12HOS and Δ12LOR common part in RHA 345 HO line (SEQ N1) was cloned, sequenced and characterized. It was shown that it carries a putative functional Δ12-desaturase gene (SEQ N1-Δ12). Indeed, the TATAAA and CAAT consensus promoter elements are present at positions -92 bp and -42 bp upstream the +1 transcriptional point, respectively. Because of its sequence and its position, the AAGTAA sequence, 16nt before the end of the transcribed part, corresponds to a poly-A signal AATAAA, except for one nucleotide. The Δ12-desaturase gene is interrupted by a 1686pb intron between nt 83 and nt 1767 upstream the +1 transcriptional point. The consensus splicing sites GT and AG are present at the intron extremities. A 16 repeats of a TTA SSR motive is revealed in the intron sequence between nt 784 and 832 upstream the +1 transcriptional point. Using primer pairs enabling this SSR amplification (SEQ N1-1F and SEQ N1-1R), size polymorphism at this locus in a set of 42 HO and LO genotypes was tested (Table 1).

Sequence N1-1F = 5′-TTGGAGTTCGGTTTATTTAT- 3′

Sequence N1-1R = 5′-TTAGTAAACGAGCCTGAAC- 3′

PCR amplification leads to 237 / 240 / 243 bp fragments corresponding to 15 / 16 / 17 SSR repeats, respectively. In the 174 individuals segregating population already studied (*7*), a strict co-segregation between the SSR polymorphism of the HO parental line (16 motifs) and the Δ12HOS allele (EXAMPLE 2) was revealed. So, this SSR sequence displays a polymorphism tightly linked to the Pervenets mutation. Consequently, it can be used to map the locus of the Δ12-desaturase gene in sunflower genome in most of crosses. Moreover, it may be used in selection programs for fast screening of genotypes carrying the Pervenets mutation by PCR method.

The invention relates to this SSR nucleic acid sequence present in SEQ ID N1 and SEQ ID N1-Δ12. The invention also relates to sequences having a high degree of homology with sequence SEQ ID N1 or N1-Δ12 and nucleic acid fragments that can be used as PCR primers to amplify the SSR motive such as SEQ ID N1-1F and SEQ ID N1-1R leading to a PCR fragment carrying the 15, 16 or 17 TTA motives.

In order to select molecular markers corresponding to the Pervenets mutation itself, parts of the 7.9 kb *Eco*RI HO specific fragment that should carry the Pervenets mutation was cloned, sequenced and characterized. Primer pairs on both side of the 5' Pervenets mutation insertion point: SEQ ID N1-2F located on SEQ N1 and SEQ ID N2-1R located on the SEQ N2 and designed in Δ12-desaturase cDNA were selected.

Sequence N1-2F = 5'-CAAACCAACCACCCACTAAC-3'

Sequence N2-1R = 5′-AGCGGTTATGGTGAGGTCAG-3′

Because of their positions, these primers lead to a 3026bp PCR amplification fragment only in RHA 345 HO genotype compared to LO4 and other LO genotypes. Subsequently, we cloned sequenced and characterised this PCR fragments carrying the 5' Pervenets mutation insertion point (SEQ ID N1/2) was cloned, sequenced and characterized. The organisation of this sequence is, from 5' to 3':
- A 2576bp HO / LO common region
- the 5' insertion point
- HO specific Δ12-desaturase gene like sequence: part of the intron (239bp) and part of the coding region (211bp).

Based on this characterisation, new primers were computed (SEQ ID N1-3F located on SEQ ID N1, SEQ ID N2-2R and SEQ ID N2-3R designed on Δ12-desaturase cDNA sequence).

Sequence N1-3F = 5'-GAGAAGAGGGAGGTGTGAAG-3'

Sequence N2-2R = 5′-ACAAAGCCCACAGTGTCGTC-3′

Sequence N2-3R = 5'-GCCATAGCAACACGATAAAG-3'

These primer pair combinations lead to HO specific amplification fragments of about 870bp (SEQ ID N1-3F + SEQ ID N2-1R), 1000bp (SEQ ID N1-3F + SEQ ID N2-2R) and 1400bp (SEQ ID N1-3F + SEQ ID N2-3R) in PCR experiments involving 42 HO and LO genotypes (EXAMPLE 1). In the 174 recombinant inbred line population, a strict co-segregation between the SEQ ID N1-3F + N2-1R HO specific fragment and the Δ12HOS allele (EXAMPLE 2) was revealed.

The invention relates to SEQ N1/2 (SEQ ID NO:1) corresponding to part of the Δ12-desaturase HO specific fragment, and sequences having a high degree of homology with SEQ N1/2 (SEQ ID NO:1). The invention also relates to nucleic acid fragments which can be used as PCR primers to amplify genomic region having a high degree of homology with SEQ ID N1/2 such as combinations of SEQ ID N1-2F or SEQ ID N1-3F with SEQ ID N2-1R, SEQ ID N2-2R and SEQ ID N2-3R. These primer pair combinations enable to obtain fragment lengths of 3026bp (sequence N1-2F+ sequence N2-1R), 870bp (sequence N1-3F + sequence N2-1R), 1000bp (sequence N1-3F + sequence N2-2R) and 1400bp (sequence N1-3F + sequence N2-3R), respectively. Those primer sequences are coded:

Sequence-N1-2F = 5′-CAAACCACCACCCACTAAC-3′

Sequence-N1-3F = 5'-GAGAAGAGGGAGGTGTGAAG-3'

Sequence-N2-1R = 5'-AGCGGTTATGGTGAGGTCAG-3'

Sequence-N2-2R = 5′-ACAAAGCCCACAGTGTCGTC-3′

Sequence-N2-3R = 5'-GCCATAGCAACACGATAAAG-3'

As above-mentioned the invention also relates to a sequence common to LO and HO regions which is localised over the insertion point of the Pervenets mutation. This sequence SEQ ID NO:13 was isolated by RAGE PCR and the sequences of the cloned LO and HO fragments clearly displays the break-up at the insertion point. Primer pairs were defined to amplify across the insertion Pervenets mutation point.

Sequence N2-1F = 5'-TTTTACTCTTTGTTATAATAG-3'

Sequence N3- 1R = 5'-TTTTTAGTTCATGGAATCAC-3'

Sequence N2-2F = 5'-ACACTAACACTCATTACATTCG-3'

Sequence N3-2R = 5'-CCTAAAGCTCTGTAGATTTT-3'

Sequence N2-3F = 5'-AAAGCAAAAAACACCGTGATTC-3'

Sequence N3-3R = 5'-GGTGTTATTATTCAGCCTGAA-3'

The use of these primers respectively leads to :
- a fragment of 170 bp (SEQ ID N0:14 and SEQ ID N0:15)
- a fragment of 160 bp (SEQ ID NO:16 and SEQ ID N0:17)
- a fragment of 170 bp (SEQ ID N0:18 and SEQ ID N0:19)

We also demonstrated that sunflower carries also a duplicated sequence at a second locus but where there is no insertion. Thus, the use of these primer pair combinations always revealed these short fragments whatever the LO or HO genotypes. However, this region enables to further characterize revertant mutant that still carry a short insertion that cannot silence the wild desaturase gene, since these were still LO.

Combination of primers amplifying the SSR, the Δ12HOS allele and the sequence across the insertion site will boost improvement of sunflower with high oleic oil content.

Troubles faced by breeders to improved HOAC sunflower are mainly due to the instability of the Pervenets mutation that seems to disappear for two reasons:
1) There is a reversion, corresponding to a deletion in the insertion (several have been characterized) that may lead to the LO phenotype when the silencing mechanism is stopped.
2) There is a suppressor that prevents the silencing mechanism and consequently, the phenotype is LO.

For the reversion the combination of the markers (SSR, Δ12HOS allele and that across the insertion site) enables to unravel what happened. Since there is no genetic recombination between the SSR locus and the Δ12HL locus, the SSR allele will be always linked (in the same phasis) to the Δ12HL allele that was present. If it reverts the Δ12LOR allele will be found linked to the incorrect SSR allele.

Moreover, when the reversion leaves a small DNA fragment in the insertion site, it is possible to directly detect it with the primer pairs described that amplify the sequence over the insertion site.

Changes in the length of the insertion were found in twelve out of 174 recombinant inbred lines. Thus it is frequent and may disturb the expected frequency ratio for HO/LO.

In the progenies, ten individuals carried the SSR of the HO parent (RHA345) linked with a modified insertion. Once corrected the HO LO ratio fits the Mendelian proportion that was distorted since ten LO progenies were in fact revertant.

The suppressor is detected when the presence of the Δ12HOS allele does not lead to the HO phenotype.

### DESCRIPTION OF THE FIGURES

- FIG. 1 Δ12HOS and Δ12LOR physical maps established according to *Eco*RI and/or *Hind*III RFLP profiles revealed with the Δ12-desaturase cDNA (8).
- FIG. 2 Outcome map of the 29 kb region with SEQ positions in HO genotypes (B) and in LO genotypes (A).
- FIG. 3 HO specific PCR amplifications in a set of HO and LO genotypes using primer pair combination SEQ ID N1-3F + SEQ ID N 2-1R (A), SEQ ID N1-3F + SEQ ID N 2-2R (B), SEQ ID N1-3F + SEQ ID N 2-3R (C).

### DESCRIPTION OF MATERIALS AND METHODS

### - MATERIALS

### . Plant materials

a. Diversity analysis:
42 HO and LO genotypes to test SSR and other PCR amplification polymorphisms were selected from different public and private institutes in order to represent a wide sunflower genetic diversity. The HO RHA345 and the LO LO4 genotypes (Table 1) were used for long PCR experiments.

**Table 1:**

| List of genotypes used to test SSR and other PCR amplification polymorphisms with their pedigree, origin and phenotype. | | | |
|---|---|---|---|
| CODE | PEDIGREE | ORIGIN | PHENOTYPE HO/LO |
| LO1 | | Monsanto | LO |
| LO2 | | Monsanto | LO |
| LO3 | | Monsanto | LO |
| LO4 | | Monsanto | LO |
| LO5 | | Monsanto | LO |
| LO11 | | Monsanto | LO |
| LO14 | | Monsanto | LO |
| LO17 | | Monsanto | LO |
| LO36 | | Monsanto | LO |
| LO38 | | Monsanto | LO |
| LO40 | | Monsanto | LO |
| HA89A | VNIIMK8931 | Russia | LO |
| BD70080 | | Monsanto | LO |
| HO1 | | Monsanto | HO |
| HO2 | | Monsanto | HO |
| HO5 | | Monsanto | HO |
| HO9 | | Monsanto | HO |
| HO19 | | Monsanto | HO |
| HO22 | | Monsanto | HO |
| HO24 | | Monsanto | HO |
| HO26 | | Monsanto | HO |
| HO37 | | Monsanto | HO |
| HO39 | | Monsanto | HO |
| HO41 | | Monsanto | HO |
| HO42 | | Monsanto | HO |
| HO43 | | Monsanto | HO |
| OPA1 | PAC2 x RHA344 | INRA - France | HO |
| R-OL1 | | Cordoba | HO |
| OPA2 | PAC2 x RHA344 | INRA - France | HO |
| BE73201-1 | | Monsanto | HO |
| BE-73201-4 | | Monsanto | HO |
| BE-73201-5 | | Monsanto | HO |
| RHA345 | | USA | HO |
| RHA346 | | USA | HO |
| RHA347 | | USA | HO |
| LG26 | | Russia | HO |
| S1 Pervenets | VNIIMK 8931 | Russia | HO |
| S1 Pervenets | VNIIMK 8931 | Russia | HO |
| S1 Pervenets | VNIIMK 8931 | Russia | HO |
| RHA 345 | | USA | HO |
| VNIIMK 8931 | | Russia | LO |

b. Segregating recombinant inbred line population:
The (LO) line 83HR4 (INRA), male-sterilized by gibberellin, was crossed with the (HO) line RHA345 (USA) in the INRA nursery during the summer of 1996. Nine F₁ hybrid seeds were obtained and the F₁ plants were inter-crossed to produce a F₂ generation in a greenhouse during the following winter. 174 F₆ progenies were obtained from these F₁ plants. These progenies were used to determine 18:1 content separately on half of a cotyledon of five seeds for each F₆ family. These seeds were sown in Jiffypots and after 6 days in a greenhouse they were transferred to the field. For each F₆ family, plant number 2 in the field was selected for molecular characterizations (RFLP and PCR genotyping). 83HR4 and RHA345 parental lines were included as controls.

### Probes

Δ12-desaturase cDNA used in the following example has 1458 bp and is similar to the complete Δ12-desaturase cDNA deposited in the GenBank under n° U91341 and described by Hongtrakul et al (5).

### - METHODS

### . 18:1 oleic acid measurement

Oil composition measurement and 18:1 content determination were performed on half a cotyledon using gas chromatography as described by Conte et al. (10).

### . Extraction of the genomic DNAs.

The DNA was extracted from 5g of ground leaves in liquid nitrogen according to the method disclosed by Gentzbittel (*11*).

After the addition of 9 ml of a sodium sulphate solution (28 mg/ml) prepared in the buffer CTAB 2X (CTAB 2% (w/v), Tris-HCl 10mM pH8, Na₂ EDTA 100 mM pH8, NaCl 1.4 M, PVP 1% (w/v)), the mixture was incubated at 65°C for 30 minutes. Five ml of chloroform/isoamylic alcohol (24/1, v/v) was added before centrifugation at 10.000 rpm, 10 min. The supernatant was recovered and incubated at 37°C for 30 min after the addition of 500 mg RNase A, then for one hour also at 37°C after the addition of 4 mg of proteinase K. One ml of CTAB 10X (CTAB 10% (w/v), NaCl 0.7 M) and 7 ml of chloroform/isoamylic alcohol (24/1, v/v) was added before centrifugation at 10.000 rpm, 10 min. The supernatant was recovered in a precipitation buffer, the volume of which corresponds to two volumes of the supernatant (CTAB 1% (w/v), Tris-HCl 50mM pH8, EDTA 100mM pH8).

After centrifugation at 12.000 rpm, for 15 min., the pellet was recovered and dissolved in 3 ml of TE High buffer (Tris-HCl 10 mM pH8, Na₂ EDTA 1mM pH8, NaCl 1M) and two volumes of 95% cold ethanol, before a further centrifugation at 12,000 rpm, for 15 min. The pellet containing the DNA was dissolved in 1 ml of TE 0.1X (Tris-HCl 1 mM pH8, Na₂ EDTA 0.1 mM).

The amount of DNA is determined using an absorbance spectrophotometer (DO) at 260 nm.

### . RFLP TECHNIQUES

### Southern blot

Eight µg of DNA of each genotype were restricted with *Eco*R*I* and/or *Hind*III enzymes according to the provider recommendations (Boehringer). Eight enzyme units per µg of DNA were used and each restriction reaction was carried out at 37°C for 6 hours.

The restriction products were separated by gel electrophoresis on Agarose 0.8% (w/v) gel in the 0.5X TBE buffer (Tris-HCl 45 mM pH8, boric acid 45mM, EDTA pH8 1mM) at 1 v/cm for 16 hours. The gel was then colored in EtBr bath (1 µg/ml). The migration profiles were made visible under UV light (at 312 nm).

After 30 min of partial depurination of the DNA by a 0.25 N HCl solution, the samples were transferred onto a Nylon membrane (Appligene). The transfer was made under reduced pressure at 60 mbar during 2 hours using a vacuum transfer apparatus (Appligene). A 0.4 N NaOH solution was used as a transfer solution. The membranes were then washed in a 2X SSC solution (NaCl 3M, sodium citrate 0.3 M pH 7). The DNA was fixed on the membranes by incubation at 80°C for two hours. The membranes were stocked at 4°C until using them for hybridization.

### Hybridization

The radioactive labelling of the probes was carried out by the random primer elongation in the presence of a desoxyribonucleotide labelled by a radioactive isotype, α³²P-dCTP according to the method described by Feinberg and Volgelstein (*12*). The prehybridization and the hybridization were carried out in the same buffer containing SDS 7% (w/v), Sodium Phosphate buffer 0.5 M (pH 7.2), EDTA 1mM (pH 8), stirring at 62°C for 1 hour (prehybridization) and 12 hours (hybridization). Two washes for 10 min. at 60°C were carried out using a buffer containing SDS 1% (w/v), Sodium Phosphate buffer 40mM (pH 7.2), EDTA 1mM (pH 8) according to methods described by Sambrook et al. (*13*). The conditions of Na⁺ molarity and of temperature determine stringence conditions high enough to ensure specific hybridizations. An autoradiographic film was then exposed to the membrane at -70°C for a duration, which depends on the intensity of the radioactive signals emitted by the membrane.

### . Specific PCR amplification

Each PCR amplification was carried out starting from 80 ng of genomic DNA in a final volume of 30 µl containing Tris-HCl 10mM (pH 8.3); KCI 50 mM, MgCl₂ 1.5 mM; dNTPs 200 µM; 1 µM of each primer, Taq polymerase 1U (Sigma). The amplifications were carried out using a PTC 100 thermocycler (MJ Research) according to the following program:
- denaturation: 94°C; 4 min.
- production (35 cycles): 94°C; 1 min.
   55°C; 1 min.
   72°C; 1 min.
- final elongation: 72°C; 5 min.

The amplification products were then either separated by electrophoresis on Agarose gel or purified in order to be cloned and sequenced. The electrophoresis was carried out on 1.2% (w/v) Agarose gel in TBE 0.5X buffer (10v/cm) for two hours. Purification of the amplification products was carried out with the Wizard PCR Prep DNA Purification Systems kit (Promega).

### . Amplification of SSR fragments.

The amplification using the SSR primers was carried out using 40 ng of genomic DNA in a final volume of 25 µl containing Tris-HCl 10 mM (pH 8.3), KCI 50 mM, MgCl₂ 1.5 mM, dNTPs 200 µM, 1 µM of each primer; Taq polymerase 1U (Sigma). The amplifications were carried out using a PTC 100 thermocycler (MJ Research) according to the following program:
Denaturation: 94°C; 5 min.
- production (35 cycles): 94°C; 30 s.
   50°C; 1 min.
   72°C; 1 min.
- final elongation: 72°C; 5 min.

Amplification product were loaded onto 6% denaturing polyacrylamide gels containing 7.5 M urea, 6% acrylamide and 1X TBE buffer (Tris-HCl 90mM pH8, boric acid 90 mM, EDTA pH8 2mM). Gels were run in a 1X TBE buffer for 90 min at 60V. SSR amplifications were visualized by sliver staining with a commercial kit from Promega.

### . Long PCR amplification

In order to amplify PCR fragment from genomic DNA longer than 2 kb, we used the Expend Long Template PCR System from Roche Applied Science. The long PCR were made according to the provider instructions.

### EXAMPLE 1: Diversity analysis with the HO specific PCR fragment across the insertion Pervenets point.

The Pervenets mutation was labelled by HO specific PCR amplifications across the 5' insertion point using designed primer pair combinations (SEQ N1-3F located on SEQ N1 with SEQ N2-1R, -2R or -3R designed on Δ12-desaturase cDNA sequence).

sequence N1-3F = 5′-GAGAAGAGGGAGGTGTGAAG-3′

sequence N2-1R = 5'-AGCGGTTATGGTGAGGTCAG-3'

sequence N2-2R = 5′-ACAAAGCCCACAGTGTCGTC-3′

sequence N2-3R = 5′-GCCATAGCAACACGATAAAG-3′

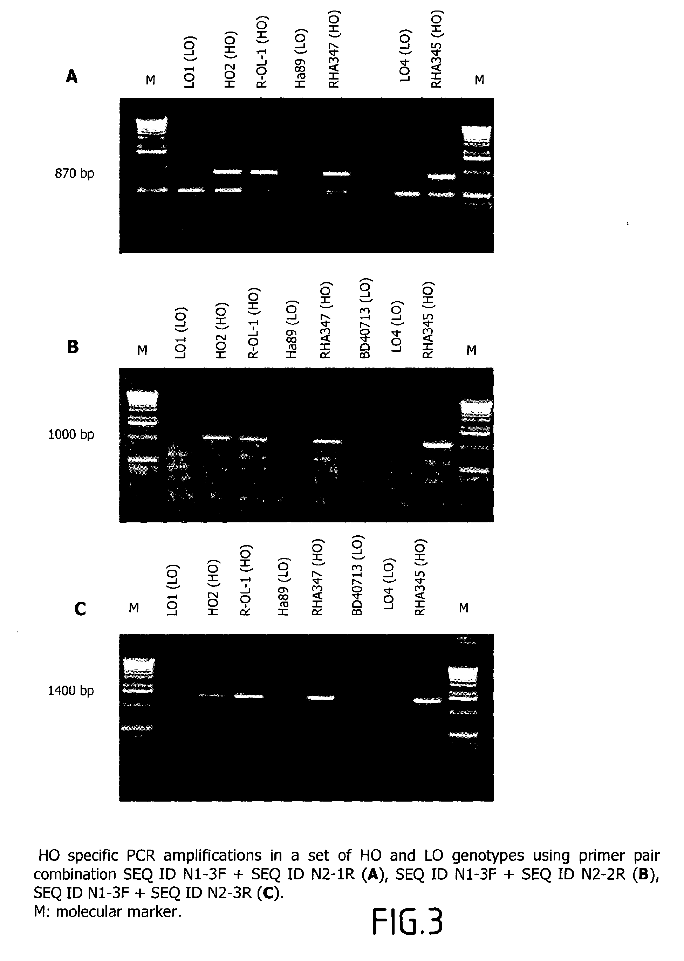

Forty-two HO and LO genotypes were used for these PCR experiments. These genotypes were previously genotyped using Δ12-desaturase cDNA as a probe to reveal RFLPs. All the HO genotypes displayed the Δ12HOS allele whereas the LO genotypes displayed the Δ12LOR one (*6*). PCR amplification products of about 870bp (SEQ ID N1-3F + SEQ ID N2-1R), 1000bp (SEQ ID N1-3F + SEQ ID N2-2R) and 1400bp (SEQ ID N1-3F + SEQ ID N2-3R) were specifically revealed in HO genotypes carrying the Pervenets mutation (Fig 3 A, B and C, respectively). All the 42 genotypes were categorized into LO or HO without any error according to these HO specific PCR amplifications.

### EXAMPLE 2: Pervenets mutation labelling in the recombinant inbred line population

The recombinant inbred line families were obtained by crossing the lines 83HR4 (LO) by RHA 345 (HO) according to the method described above. Using Δ12-desaturase as a probe to reveal RFLP, we showed that the RHA 345 HO parent line carries the Δ12HOS allele whereas the 83HR4 LO parental line displayed the Δ12LOR allele. In the 174 recombinant inbred line population, the Δ12HOS / Δ12LOR alleles segregated as 78 / 96.

The Pervenets mutation was labelled by the 870bp PCR fragment across the 5' insertion point and by the polymorphism of the SSR locus located on the Δ12-desaturase gene intron. Moreover this SSR polymorphism labelled the Δ12-desaturase gene itself.

870bp HO specific PCR amplification: We used designed primer pair combination SEQ ID N1-3F with SEQ ID N2-1R:

sequence N1-3F = 5'-GAGAAGAGGGAGGTGTGAAG-3'

sequence N2-1R = 5′-AGCGGTTATGGTGAGGTCAG-3′

In the 174 recombinant inbred line population, it leads to PCR amplification of about 870 bp only in genotypes carrying the Δ12HOS allele and thus the Pervenets mutation.

Amplification of the SSR sequence: The SSR polymorphism between RHA 345 and 83HR4 parental line was revealed by PCR amplification using the primer pair SEQ ID N1-1F/ SEQ ID N1-1R. This PCR amplification leads to 237 bp (15 SSR motives) and 240 bp (16 SSR motives) in 83HR4 and RHA 345, respectively. The segregation of this SSR polymorphism was tested in the 174 recombinant inbred line population. All the genotypes carrying the Δ12HOS allele displayed the HO RHA 345 SSR polymorphism (16 SSR motives) whereas genotypes carrying the Δ12LOR allele displayed the LO 83HR4 SSR polymorphism (15 SSR motives).

PCR methods with these primer pairs enabling to amplify either the Pervenets mutation itself or the SSR locus, lead to distinguish between genotypes carrying the Pervenets mutation and genotypes without the mutation. Consequently, the SSR sequence and the HO PCR specific fragment may be used in selection programs to identify genotypes carrying the Pervenets mutation. Moreover, the SSR polymorphism can be used to map the Δ12-desaturase gene.

### REFERENCES

(1) Soldatov KI. (1976). Chemical mutagenesis in sunflower breeding. In *Proc. VII*^{*th*} *Int. Sunflower Conference,* June 27^{th} - July 3^{rd}, 1976, Krasnodar.
(2) Garcès R, Garcia J, Mancha M. (1989). Lipid characterization in seeds of a high oleic acid sunflower mutant. Phytochemistry 28 (10): 2597-2600.
(3) Garcés R et Mancha M. (1991). *In vitro* oleate desaturase in developping sunflower seeds. *Phytochemistry30:2127-2130.*
(4) Kabbaj A. et al. (1996). Polymorphism in *Helianthus* and expression of stearate, oleate and linoleate desaturase genes in sunflower with normal and high oleic content. *Helia* 19:1-18.
(5) Hongtrakul V, Slabaugh MB, Knapp SJ (1998). A seed specific □12 oleate-desaturase gene is duplicated, rearranged and weakly expressed in high oleic acid sunflower lines. *Crop Science* 38:1245-1249
(6) Lacombe S et Bervillé A (2001). A dominant mutation for high oleic acid content in sunflower *(Helianthus annuus* L.) seed oil is genetically linked to a single oleate-desaturase RFLP locus. *Molecular Breeding* 8:129-137.
(7) Lacombe S et al. (2001). An oleate-desaturase and a suppressor locus direct high oleic acid content of sunflower *(Helianthus annuus* L.) oil in the Pervenets mutant. *C.R. Acad. Sci, Sciences de la vie* 324:839-845.
(8) Lacombe et al. (2002) Genetic, molecular and expression feature of the Pervenets mutant leading to high oleic acid content of seed oil in sunflower. *OCL* 9:17-23
(9) Devereux, Haeberli and Smithies (1984). A Comprehensive Set of Sequence Analysis Programs for VAX. Nucleic Acids Research 12(1); 387-395
(10) Conte et al. (1989) Half seed analysis: rapic chromatographic determination of the main fatty acids of sunflower seeds. Plant Breeding 102:158-165
(11) Gentzbittel et al. (1994) RFLP studies of genetic relationships among inbred lines of cultivated sunflower *(Helianthus annuus* L.): evidence of distinct restorer and maintener germplasm pools. *Theor. App. Genet* 89:419-425.
(12) Feinberg AP et Vogelstein B. (1983). A technic for radiolabelling DNA restriction endonuclease fragments to high specific activity. Analytical Biochemistry 132: 6-13.
(13) Sambrook J, Fritsch EF, Maniatis T. (1989). Molecular cloning: a laboratory manual (2^{nd} edn). Cold Spring Harbor Laboratory Press, Cold Spring Harbor, NY.

## Claims

1. Isolated nucleic acid having the sequence SEQ ID NO:3.

2. Isolated nucleic acid fragments having 10-30 bp and which hybridize with SEQ ID NO:2.

3. Isolated nucleic acid fragments according to claim 3, which are selected from :

sequence N2-1R: 5'-AGCGGTTATGGTGAGGTCAG-3' (SEQ ID N°7)

sequence N2-2R:      5'-ACAAAGCCCACAGTGTCGTC-3' (SEQ ID N°8)

sequence N2-3R:     5'-GCCATAGCAACACGATAAAG-3' (SEQ ID N°9)

4. Isolated nucleic acid fragments having the sequence SEQ ID NO:11 or SEQ ID NO: 12.

5. Primer pairs useful for nucleic acid amplification, which comprise :
1) as the first member of the pair a nucleic acid fragment having 10 to 30 bp and which hybridises with sequence N1 (SEQ ID N°1) and
2) as the second member of the pair a nucleic acid fragment having 10 to 30 bp and which hybridises with SEQ ID NO:2.

6. Primers pairs according to claim 5, which comprise as :
1) first member : sequence N1-2F : 5'-CAAACCAACCACCCACTAAC-3' (SEQ ID N°5) or sequence N1-3F : 5'-GAGAAGAGGGAGGTGTGAAG-3' (SEQ ID N°6)
2) second member :

sequence N2-1R:	5'-AGCGGTTATGGTGAGGTCAG-3' (SEQ ID N°7)

sequence N2-2R: 5'-ACAAAGCCCACAGTGTCGTC-3' (SEQ ID N°8)

sequence N2-3R: 5'-GCCATAGCAACACGATAAAG-3' (SEQ ID N°9)

7. Isolated nucleic acid having the sequence SEQ ID NO:13.

8. Isolated nucleic acid fragments having 10-30 bp and which hybridize with SEQ ID NO:13.

9. Isolated nucleic acid fragments according to claim 8, which are selected from :

sequence N2-1F: 5'-TTTTACTCTTTGTTATAATAG-3' (SEQ ID N°14)

sequence N2-2F: 5'-ACACTAACACTCATTACATTCG-3' (SEQ ID N°16)

sequence N2-3F: 5'-AAAGCAAAAACACCGTGATTC-3' (SEQ ID N°18)

10. Isolated nucleic acid fragments according to claim 8, which are selected from :

sequence N3-1R: 5'-TTTTTAGTTCATGGAATCAC- 3' (SEQ ID N°15)

sequence N3-2R: 5'-CCTAAAGCTCTGTAGATTTT- 3' (SEQ ID N°17)

sequence N3-3R: 5'-GGTGTTATTATTCAGCCTGAA- 3' (SEQ ID N°19)

11. Process for selecting sunflower having seeds with a high content of oleic acid which comprises the steps of:
- extracting the genomic DNA;
- amplifying said genomic DNA by means of primer pair, said pair being constituted with a first member of nucleic acid fragment having 10 to 30 bp which hybridises with sequence N1 (SEQ ID NO:1) and a second member of nucleic acid fragment having 10 to 30 bp which hybridises with sequence N2 (SEQ ID NO:2);
- hybridizing the amplified DNA fragment with the labelled sequence SEQ ID N° 3 and
- isolating the genotypes giving a positive hybridization signal.

12. Process for mapping the Pervenets mutation on the genome, which comprise the steps of:
- extracting the genomic DNA;
- amplifying said genomic DNA by means of primers SEQ ID N° 11 and SEQ ID N° 12;
- isolating the clones having a DNA fragment length which is different from a reference clone without the Pervenets mutation.

13. Process for amplifying the region across the insertion Pervenets mutation site both in LO and HO genotypes, which comprises the steps of :
- extracting the genomic DNA;
- amplifying said genomic DNA by means of primer pair, said pair being constituted with a first member of nucleic acid fragments having 10 to 30 bp which hybridizes with sequence N1 (SEQ ID NO: 13).

14. Combination of primers comprising at least a primer pair according to claims 5 and 6, at least a primer pair selected from the nucleic acid fragments as defined in claims 8-10 and at least a primer pair having the sequence SEQ ID NO:11 and the sequence SEQ ID NO: 12.

15. Test kits for selecting sunflower having seeds with high content of oleic acid comprising at least a primer pair according to claims 5 and 6, at least a primer pair selected from the nucleic acid fragments as defined in claims 8-10 and at least a primer pair having the sequence SEQ ID NO:11 and the sequence SEQ ID NO:12.


SEQUENCE LISTING

<110> INSTITUT NATIONAL DE LA RECHERCHE AGRONOMIQUE

MONSANTO SAS

<120> Method of selecting sunflower genotypes with high oleic acid content in seed oil

<130> 1H13806-5EP

<160> 19

<170> PatentIn version 3.1

<210> 1

<211> 6662

<212> DNA

<213> Helianthus annuus

<400> 1
aagcttaatt tataaagttt gagtttggta cgtgaatagt ttttaaacat ttactcaata 60
agtgaaactt gggcctaggc tcatttatta tttataattt atttcattaa tttatttata 120
aacaattatt gaagcttggg tctggactcg tttattaaac aaaccatttt tagctcgacc 180
tcgtttaagt ccgacttgat cgagctttta atgactcaat cttgggtagc tcacaagcga 240
ctcatatcat ttagccccta acttaatgct ttggaacaga aaatgttaga ccatgtgtta 300
tcgtctcaca aattttaacc accagtcata cggtagctcc tacatcactc acacatcatc 360
agaccatttt ctttcatttt tttcactgca cctcatagta atgttactgg ttttaaccat 420
ccaccatcca ttaaaataat atttaaatta tataagctaa aaatctttga tcggttgctt 480
gccgacttgg tatttttttcc atggtagcaa atgaaatatt cctccaaggt gaaccggcta 540
acgcggttct cccttttaca gtcggtaacg ccccttaaga cagtccgatg gccagtgatg 600
tagccggggc gttaccttgt aacacatagc gttacaacaa gcaaccttta aaacacttgt 660
ttgaaatcgg tgaatgtttg tttgtttctt gtggcacctt taaaacactt gtcctttaaa 720
acactatcgg tgacaagtgt tctctgacca aggtcctttc gtccaatatt caaaacctca 780
gggacacttt atgaaaaata ttctctctat atataaaccg ctaaaccagc aacgctttct 840
gtgaacggaa gcacatcgtc gtctgtctca ctagcggaga ctcaccgttt ctacgaagtg 900
aaaaccctaa gcctctgtcg ctcgatcgat cgatcgattc aggtattgtt tgaatcatcg 960
catttcgata aatcattaat tatcgaattt tgcttttgat ttttaattta atttacttag 1020
ctgctgcatt tatgattgat gaacagatca gttgatttct cttctctgtg actttgattt 1080
ggttcatttc actcagtttc ctttaaattt gtgcgtttat ctgtcttgca tgtgatcgat 1140
tttgcttcct tcatgtgttt tcattcaata tgtactcttt taacttgtaa aatcttgcct 1200
gattgatttt cttcttgatt tttgtgatca actctttaag tttttttttt tttacaagat 1260
tttctgtttt agtcgagtca taaaagtgcg taaatcattg acaggtgtca attgtttatt 1320
tttttttctg gtagtaataa taaaagcatc agcttttata ttgggttgat gtattttcgg 1380

taaatgtttt gcgttaaagc taaagaaaca tgaatttcat ggcttttgta atcaagtgta 1440
aattgacgac tagatctacg aattatttct atttatttta aaacaaaatc gttaaataca 1500
aaaataaata gtgagttcat ttatcgttaa atacaaaaat aaatagtgag ttcattttat 1560
gcatgtttta tcctagggtg aaaacggaga cgagccgcga gttggagttc ggtttattta 1620
taatttatta attagttatt atgattatta ttattattat tattattatt attattatta 1680
ttattattat tattttgtat ataatttagt aatttttcat aattttatat gtagtaatta 1740
ttattattta aatatatatt ttatatatca aattaaatat tgtataaccg atgggcttgt 1800
ttaggctcgt gagcgagctc gagttcaggc tcgtttacta aacaagctca cctttagact 1860
cgggcttcaa ctcgtttaag cctggctcgt tagagctttt tttttctagc tcggctcatt 1920
ttcaccctta ttttatcctt tagaccagac tcagattgtt ttttaacgtt aaatccactc 1980
atatgccaat aacatttggg caaaaacgca ttatgtctaa acacgttttt taaaacatta 2040
ttctttcaaa acatttttag ataatataaa accatgtgta gtaagtagta cacatgaata 2100
atgcccccat caatgggcgt tgtgcgacac gtgtcatccc agtcagcaaa ggggcattat 2160
gggggttttt catatatggg tgtgggacaa tgccccatca atgattaccc aattattatc 2220
ttttaaaaca aataatatta cttggaagct tcttattgga caaataaata atgccccagc 2280
gtttagacca cacccccccc cccctttctg aaaacaacgc ccaagggggg cagtctggcg 2340
gcgttaaggg gcgttttttt ggggaaaaac acccaaaaac ccccagtacg ggtggtctaa 2400
gcataaaatg tgtttacaat atattttaaa aaacactgat ttaatggaga caacatttaa 2460
gacatactgt catataatat tattatacaa aagtagtaat tagtaatcgt gttacgtaat 2520
ataatgtgta tttgatagtc tcgtatctac tttttatgta tctatttcat aaagaaaagg 2580
ttaggcatgt ttgtttgtaa atataaagat attgttcttg cttgcaggtt gaaaagtctg 2640
gtcaaacagt caacatatgg gtgcaggaga atacacgtct gtgaccaacg aaaacaaccc 2700
actcgatcga gtccctcatg caaaaccacc cttcaccatc ggcgatctga aaaaagccat 2760
cccaccacac tgcttccagc ggtcgctaac ccgttcgttc tcctacgtgc tgtctgacct 2820
caccataacc gctgtcctct accacattgc caccacctac ttccaccacc tccccacccc 2880
tttgtcatcc atcgcatggg cctcttactg ggtagtccaa ggctgcgtcc tcaccggagt 2940
ctgggtcatc gcccacgaat gtggtcacca tgcgtttagt gattatcaat gggtcgacga 3000
cactgtgggc tttgttctcc actcgtcttt actcgtccct tacttttcgt ggaaatatag 3060
tcaccaccgc caccattcca acactggatc gastcgagcg ggacgaggtt ttcgtcccca 3120
aatcccgatc gaaagtcccg tggtactcga aatactttaa caacacagtg ggccgcattg 3180
tcagtatgtt cgtcactctc actctcggct ggcccttgta cttagctttc aatgtgtcgg 3240
gccgacccta tgaccgtttc gcctgccact acgtcccaac cagccctatg tacaatgaac 3300
gtaaacgtta ccagatagtc atgtccgaca tcgggattgt tatcacatcg ttcatccttt 3360
atcgtgttgc tatggcaaaa gggttggttt gggtgatttg cgtctatggg gttccgttga 3420
tggttgtgaa cgcgtttctg gtgttgatca cttatcttca acatactcac cctggcttgc 3480
cgcattatga tagctcggaa tgggaatggt taaagggagc attggcgaca gtggaccgtg 3540
actatggtgt gttgaacaag gtgttccatc atattaccga cacacatgtg gtgcaccatt 3600
tgttttcgac aatgcctcat tataatgcga tggaagcaca gaaggcgctg agaccggtgc 3660
ttggggagta ttatcggttt gacaagaccc cgttttatgt agccatgtgg agagagatga 3720
aggaatgttt gtttgtggag caagatgatg aagggaaagg aggtgtgttt tggtacaaga 3780
ataagatgaa ttaataagtg tttagtggat gtctatgctt attaagtctg tgacaatggg 3840
tcttgtcctg gttctgggtc tgggtctggt tagtgtgtgt ggttagtgtg tgttgtgggt 3900
caagtgtatg tcagatggat gtaacgcagt atgtgttgcc gtgttgggtg tgttttagaa 3960
ctattaatga atatattata tatatctctt atttgggtga actcwamtcr rgywatgsrt 4020
yyaamtgsgy trgsascwct cmcgactatk gtykayctkc asgcrkcyrc kwrtymacka 4080
gtgmytcaaa ccaccaccca ctaacaacgt tacatcacca ccgtagtcac cgccaccttc 4140
acttgtcgcc actagcctcc ggccaccgta gtcaccgcca tcttcaccca tcgccaccag 4200
cctccgtaag atgagatatg aaggatgaac ttaaattgat tgtatttttt cttaaacgat 4260
catttcaaac tccattttag caagagccta tttgattcaa accaaattga tattttttac 4320
acagctcgtt ttgacctgaa ctcttgccca gaacctgttt tgacgtgaac ctattttacc 4380
tgccgattag cagtattgac ggagccatga gtttccttta ggaattctca aggttaaaag 4440
aaaaaagaga ttataaaaac aacaaaaatg aagattgaag aaacaacttg ctacatattt 4500
gacagacgac ataagcaaaa ttttactagc aaaacggtat atgaactcaa acgaacataa 4560
cacatccttc gggttctaca accaaagcaa aatttaacta gcagacggta tattgtatat 4620
gaacacaaat gaacgtagga cctccttcga gttctacaac caagatcaca catacatagt 4680
aattgtacaa tgcaaggggg acaagatcaa accaatcgaa aagggaagat ctgtggtagt 4740
ctattaacaa atatacaaac cgggtcccca taaacaacca taaaacattg aaactaccaa 4800
agcaagggtc atgggcgatt agccacgtat gtaccttttg tgcgtcaagt accataacca 4860
atgacggaaa accatgctag ttataaggat atgaagcaaa tcatgggatg tatgatcata 4920
atcagaatgt caaaagatca ctaatcttcc tgagagatta atgcatacaa ttctttagca 4980
gtccaacttt cttcaagaag ctcactgaga ggagccgcaa tctcattcgc cattgtcaac 5040

atgatgttca tgcaatctgt agccattcac agttcatctt aatcaacatg ttcaaatttt 5100
taatattttt atgtaatata aaaggaaatg ctaacctgga attttggtaa aattaaggag 5160
agatgtggca gcaagaatct tgtgctctac ccatccacta ttttcgaggt tttcttttag 5220
tttagagata agaaccgaga aagcagaaag ctggaactcg gactcaggta aagaagcaag 5280
tgaagaactg aaccacacca tcgtgatcra gyatactttc gtcatttcac gatgtccaca 5340
actcaagcac tgggaaaatg aatccagaaa cgagtttttc ccatcactaa caagagacgt 5400
tgacaatctg atcaaccaat cttcaacagc ctcttcctcc tcagtatcct ataagccata 5460
gcatttggtt ttagagcgct ttttatagat tataaaatta caatttaaat ccgtttaatt 5520
tagctaatct ttaataacat atttttttat agaacattaa aacgtttaac taagttactt 5580
ttaaatctcc aaaaagtaac aagaaaatat atatgttttt ttctataata ctaattgcga 5640
cagtaatggg ctttagttat gttttatttg taaaagaaat cccataattt atagtgtaaa 5700
gtacacaaat ggtccctatg gttgaccaaa attttggatt tggtctctag ctttccaaaa 5760
ctacatgaat ggtccctgtg gtttgcactt tgtaatgcat ttagtcccta gctaacaagt 5820
cttaaggttt tcgcaggtct aggttattga gtaaggtgct gtttgttttt gcagacataa 5880
attgtctgca agctgatttc atctgttttt atgtctgcaa ctgaagatgt ggtctgaaga 5940
tctgcaagct gaaaatataa gactgtttgt ttttataagt tgataccggt ctgaagcgca 6000
tcaaacaaag acgaaacatt cacattcagc ttcgatccag tggaggagaa gagggaggtc 6060
ggagaaggag ctggtcaccg gaggaggaag gaggtcgccg gaggaggagg gaggtcggag 6120
aagaggtagg tcgccggagg aggtaggtca ccggaggaga agagggaggt cgccggagga 6180
gaagagggat gtcggagaag gaggaggagc ggtggtggga ggggctgatg tttgggagag 6240
gagaagaggg aggtgtgaag atgttttaag aaaggggtcc atagcttatt ttttaagatg 6300
aaaaaagctg agttcagatc tgtttgaaaa aaaaaatcaa acagtcttca agggtaacgt 6360
ctgcgcgctt gcagacatca gcccccttga agatgtttga agaaaaaaca aacaccccct 6420
aaatgtgtta caaagtgcaa accacaggga ccatccatgt acttttgaaa agctagggac 6480
caaatccaaa attttggtta agcataggga ccatccgtgt attttactct ttgttataat 6540
aggtagagga taatacaaaa aagaatactc atagtaaaac actaacactc attacattcg 6600
tacaaactca ttacactgtc cattccgttc ctttctggtt tcgttaaata taaataaagc 6660
aa 6662

<210> 2

<211> 3397

<212> DNA

<213> Helianthus annuus

<400> 2
cctttaaaac acttgtttga aatcggtgaa tgtttgtttg tttcttgtgg cacctttaaa 60
acacttgtcc tttaaaacac tatcggtgac aagtgttctc tgaccaaggt cctttcgtcc 120
aatattcaaa acctcaggga cactttatga aaaatattct ctctatatat aaaccgctaa 180
accagcaacg ctttctgtga acggaagcac atcgtcgtct gtctcactag cggagactca 240
ccgtttctac gaagtgaaaa ccctaagcct ctgtcgctcg atcgatcgat cgattcaggt 300
attgtttgaa tcatcgcatt tcgataaatc attaattatc gaattttgct tttgattttt 360
aatttaattt acttagctgc tgcatttatg attgatgaac agatcagttg atttctcttc 420
tctgtgactt tgatttggtt catttcactc agtttccttt aaatttgtgc gtttatctgt 480
cttgcatgtg atcgattttg cttccttcat gtgttttcat tcaatatgta ctcttttaac 540
ttgtaaaatc ttgcctgatt gattttcttc ttgatttttg tgatcaactc tttaagtttt 600
ttttttttta caagattttc tgttttagtc gagtcataaa agtgcgtaaa tcattgacag 660
gtgtcaattg tttatttttt tttctggtag taataataaa agcatcagct tttatattgg 720
gttgatgtat tttcggtaaa tgttttgcgt taaagctaaa gaaacatgaa tttcatggct 780
tttgtaatca agtgtaaatt gacgactaga tctacgaatt atttctattt attttaaaac 840
aaaatcgtta aatacaaaaa taaatagtga gttcatttat cgttaaatac aaaaataaat 900
agtgagttca ttttatgcat gttttatcct agggtgaaaa cggagacgag ccgcgagttg 960
gagttcggtt tatttataat ttattaatta gttattatga ttattattat tattattatt 1020
attattatta ttattattat tattattatt ttgtatataa tttagtaatt tttcataatt 1080
ttatatgtag taattattat tatttaaata tatattttat atatcaaatt aaatattgta 1140
taaccgatgg gcttgtttag gctcgtgagc gagctcgagt tcaggctcgt ttactaaaca 1200

agctcacctt tagactcggg cttcaactcg tttaagcctg gctcgttaga gctttttttt 1260
tctagctcgg ctcattttca cccttatttt atcctttaga ccagactcag attgtttttt 1320
aacgttaaat ccactcatat gccaataaca tttgggcaaa aacgcattat gtctaaacac 1380
gttttttaaa acattattct ttcaaaacat ttttagataa tataaaacca tgtgtagtaa 1440
gtagtacaca tgaataatgc ccccatcaat gggcgttgtg cgacacgtgt catcccagtc 1500
agcaaagggg cattatgggg gtttttcata tatgggtgtg ggacaatgcc ccatcaatga 1560
ttacccaatt attatctttt aaaacaaata atattacttg gaagcttctt attggacaaa 1620
taaataatgc cccagcgttt agaccacacc cccccccccc tttctgaaaa caacgcccaa 1680
ggggggcagt ctggcggcgt taaggggcgt ttttttgggg aaaaacaccc aaaaaccccc 1740
agtacgggtg gtctaagcat aaaatgtgtt tacaatatat tttaaaaaac actgatttaa 1800
tggagacaac atttaagaca tactgtcata taatattatt atacaaaagt agtaattagt 1860
aatcgtgtta cgtaatataa tgtgtatttg atagtctcgt atctactttt tatgtatcta 1920
tttcataaag aaaaggttag gcatgtttgt ttgtaaatat aaagatattg ttcttgcttg 1980
caggttgaaa agtctggtca aacagtcaac atatgggtgc aggagaatac acgtctgtga 2040
ccaacgaaaa caacccactc gatcgagtcc ctcatgcaaa accacccttc accatcggcg 2100
atctgaaaaa agccatccca ccacactgct tccagcggtc gctaacccgt tcgttctcct 2160
acgtgctgtc tgacctcacc ataaccgctg tcctctacca cattgccacc acctacttcc 2220
accacctccc cacccctttg tcatccatcg catgggcctc ttactgggta gtccaaggct 2280
gcgtcctcac cggagtctgg gtcatcgccc acgaatgtgg tcaccatgcg tttagtgatt 2340
atcaatgggt cgacgacact gtgggctttg ttctccactc gtctttactc gtcccttact 2400
tttcgtggaa atatagtcac caccgccacc attccaacac tggatcactc gagcgggacg 2460
aggttttcgt ccccaaatcc cgatcgaaag tcccgtggta ctcgaaatac tttaacaaca 2520
cagtgggccg cattgtcagt atgttcgtca ctctcactct cggctggccc ttgtacttag 2580
ctttcaatgt gtcgggccga ccctatgacc gtttcgcctg ccactacgtc ccaaccagcc 2640
ctatgtacaa tgaacgtaaa cgttaccaga tagtcatgtc cgacatcggg attgttatca 2700
catcgttcat cctttatcgt gttgctatgg caaaagggtt ggtttgggtg atttgcgtct 2760
atggggttcc gttgatggtt gtgaacgcgt ttctggtgtt gatcacttat cttcaacata 2820
ctcaccctgg cttgccgcat tatgatagct cggaatggga atggttaaag ggagcattgg 2880
cgacagtgga ccgtgactat ggtgtgttga acaaggtgtt ccatcatatt accgacacac 2940
atgtggtgca ccatttgttt tcgacaatgc ctcattataa tgcgatggaa gcacagaagg 3000
cgctgagacc ggtgcttggg gagtattatc ggtttgacaa gaccccgttt tatgtagcca 3060
tgtggagaga gatgaaggaa tgtttgtttg tggagcaaga tgatgaaggg aaaggaggtg 3120
tgttttggta caagaataag atgaattaat aagtgtttag tggatgtcta tgcttattaa 3180
gtctgtgaca atgggtcttg tcctggttct gggtctgggt ctggttagtg tgtgtggtta 3240
gtgtgtgttg tgggtcaagt gtatgtcaga tggatgtaac gcagtatgtg ttgccgtgtt 3300
gggtgtgttt tagaactatt aatgaatata ttatatatat ctcttatttg ggtgaactca 3360
aatcgggtaa tgggtttaaa tgggctagca ccactca 3397

<210> 3

<211> 872

<212> DNA

<213> Helianthus annuus

<400> 3
gagaagaggg aggtgtgaag atgttttaag aaaggggtcc atagcttatt ttttaagatg 60
aaaaaagctg agttcagatc tgtttgaaaa aaaaaatcaa acagtcttca agggtaacgt 120
ctgcgcgctt gcagacatca gcccccttga agatgtttga agaaaaaaca aacaccccct 180
aaatgtgtta caaagtgcaa accacaggga ccatccatgt acttttgaaa agctagggac 240
caaatccaaa attttggtta agcataggga ccatccgtgt attttactct ttgttataat 300
aggtagagga taatacaaaa aagaatactc atagtaaaac actaacactc attacattcg 360
tacaaactca ttacactgtc cattccgttc ctttctggtt tcgttaaata taaataaagc 420
aacagtacgg gtggtctaag cataaaatgt gtttacaata tattttaaaa aacactgatt 480
taatggagac aacatttaag acatactgtc atataatatt attatacaaa agtagtaatt 540
agtaatcgtg ttacgtaata taatgtgtat ttgatagtct cgtatctact ttttatgtat 600
ctatttcata aagaaaaggt taggcatgtt tgtttgtaaa tataaagata ttgttcttgc 660

ttgcaggttg aaaagtctgg tcaaacagtc aacatatggg tgcaggagaa tacacgtctg 720
tgaccaacga aaacaaccca ctcgatcgag tccctcatgc aaaaccaccc ttcaccatcg 780
gcgatctgaa aaaagccatc ccaccacact gcttccagcg gtcgctaacc cgttcgttct 840
cctacgtgct gtctgacctc accataaccg ct 872

<210> 4

<211> 3026

<212> DNA

<213> Helianthus annuus

<400> 4
caaaccacca cccactaaca acgttacatc accaccgtag tcaccgccac cttcacttgt 60
cgccactagc ctccggccac cgtagtcacc gccatcttca cccatcgcca ccagcctccg 120
taagatgaga tatgaaggat gaacttaaat tgattgtatt ttttcttaaa cgatcatttc 180
aaactccatt ttagcaagag cctatttgat tcaaaccaaa ttgatatttt ttacacagct 240
cgttttgacc tgaactcttg cccagaacct gttttgacgt gaacctattt tacctgccga 300
ttagcagtat tgacggagcc atgagtttcc tttaggaatt ctcaaggtta aaagaaaaaa 360
gagattataa aaacaacaaa aatgaagatt gaagaaacaa cttgctacat atttgacaga 420
cgacataagc aaaattttac tagcaaaacg gtatatgaac tcaaacgaac ataacacatc 480
cttcgggttc tacaaccaaa gcaaaattta actagcagac ggtatattgt atatgaacac 540
aaatgaacgt aggacctcct tcgagttcta caaccaagat cacacataca tagtaattgt 600
acaatgcaag ggggacaaga tcaaaccaat cgaaaaggga agatctgtgg tagtctatta 660
acaaatatac aaaccgggtc cccataaaca accataaaac attgaaacta ccaaagcaag 720
ggtcatgggc gattagccac gtatgtacct tttgtgcgtc aagtaccata accaatgacg 780
gaaaaccatg ctagttataa ggatatgaag caaatcatgg gatgtatgat cataatcaga 840
atgtcaaaag atcactaatc ttcctgagag attaatgcat acaattcttt agcagtccaa 900
ctttcttcaa gaagctcact gagaggagcc gcaatctcat tcgccattgt caacatgatg 960
ttcatgcaat ctgtagccat tcacagttca tcttaatcaa catgttcaaa tttttaatat 1020
ttttatgtaa tataaaagga aatgctaacc tggaattttg gtaaaattaa ggagagatgt 1080
ggcagcaaga atcttgtgct ctacccatcc actattttcg aggttttctt ttagtttaga 1140
gataagaacc gagaaagcag aaagctggaa ctcggactca ggtaaagaag caagtgaaga 1200
actgaaccac accatcgtga tcaagcatac tttcgtcatt tcacgatgtc cacaactcaa 1260
gcactgggaa aatgaatcca gaaacgagtt tttcccatca ctaacaagag acgttgacaa 1320
tctgatcaac caatcttcaa cagcctcttc ctcctcagta tcctataagc catagcattt 1380
ggttttagag cgctttttat agattataaa attacaattt aaatccgttt aatttagcta 1440
atctttaata acatattttt ttatagaaca ttaaaacgtt taactaagtt acttttaaat 1500
ctccaaaaag taacaagaaa atatatatgt ttttttctat aatactaatt gcgacagtaa 1560
tgggctttag ttatgtttta tttgtaaaag aaatcccata atttatagtg taaagtacac 1620
aaatggtccc tatggttgac caaaattttg gatttggtct ctagctttcc aaaactacat 1680
gaatggtccc tgtggtttgc actttgtaat gcatttagtc cctagctaac aagtcttaag 1740
gttttcgcag gtctaggtta ttgagtaagg tgctgtttgt ttttgcagac ataaattgtc 1800
tgcaagctga tttcatctgt ttttatgtct gcaactgaag atgtggtctg aagatctgca 1860
agctgaaaat ataagactgt ttgtttttat aagttgatac cggtctgaag cgcatcaaac 1920
aaagacgaaa cattcacatt cagcttcgat ccagtggagg agaagaggga ggtcggagaa 1980
ggagctggtc accggaggag gaaggaggtc gccggaggag gagggaggtc ggagaagagg 2040
taggtcgccg gaggaggtag gtcaccggag gagaagaggg aggtcgccgg aggagaagag 2100
ggatgtcgga gaaggaggag gagcggtggt gggaggggct gatgtttggg agaggagaag 2160
agggaggtgt gaagatgttt taagaaaggg gtccatagct tattttttaa gatgaaaaaa 2220
gctgagttca gatctgtttg aaaaaaaaaa tcaaacagtc ttcaagggta acgtctgcgc 2280
gcttgcagac atcagccccc ttgaagatgt ttgaagaaaa aacaaacacc ccctaaatgt 2340
gttacaaagt gcaaaccaca gggaccatcc atgtactttt gaaaagctag ggaccaaatc 2400
caaaattttg gttaagcata gggaccatcc gtgtatttta ctctttgtta taataggtag 2460
aggataatac aaaaaagaat actcatagta aaacactaac actcattaca ttcgtacaaa 2520
ctcattacac tgtccattcc gttcctttct ggtttcgtta aatataaata aagcaacagt 2580
acgggtggtc taagcataaa atgtgtttac aatatatttt aaaaaacact gatttaatgg 2640

agacaacatt taagacatac tgtcatataa tattattata caaaagtagt aattagtaat 2700
cgtgttacgt aatataatgt gtatttgata gtctcgtatc tactttttat gtatctattt 2760
cataaagaaa aggttaggca tgtttgtttg taaatataaa gatattgttc ttgcttgcag 2820
gttgaaaagt ctggtcaaac agtcaacata tgggtgcagg agaatacacg tctgtgacca 2880
acgaaaacaa cccactcgat cgagtccctc atgcaaaacc acccttcacc atcggcgatc 2940
tgaaaaaagc catcccacca cactgcttcc agcggtcgct aacccgttcg ttctcctacg 3000
tgctgtctga cctcaccata accgct 3026

<210> 5

<211> 20

<212> DNA

<213> primer

<400> 5
caaaccacca cccactaac 19

<210> 6

<211> 20

<212> DNA

<213> primer

<400> 6
gagaagaggg aggtgtgaag 20

<210> 7

<211> 20

<212> DNA

<213> primer

<400> 7
agcggttatg gtgaggtcag 20

<210> 8

<211> 20

<212> DNA

<213> primer

<400>  8
acaaagccca cagtgtcgtc                                                    20

<210>  9

<211>  20

<212>  DNA

<213>  primer

<400>  9
gccatagcaa cacgataaag                                                    20

<210>  10

<211>  45

<212>  DNA

<213>  primer

<400>  10
ttattattat tattattatt attattatta ttattattat tatta                        45

<210>  11

<211>  20

<212>  DNA

<213>  primer

<400>  11
ttggagttcg gtttatttat                                                    20

<210>  12

<211>  19

<212>  DNA

<213>  primer

<400>  12
ttagtaaacg agcctgaac                                                     19

<210> 13

<211> 763

<212> DNA

<213> Helianthus annuus

<220>

<221> misc_feature

<222> (696)..(696)

<223> n is a, g, c or t

<400> 13
cactaacact cattacattc gtacaaactc attacactgt ccattccgtt cctttctggt 60
ttcgttaaat ataaataaag caacagtacg ggtggtctaa gcataaaatg tgtttacaat 120
atattttaaa aaacactgat ttaatggaga caacatttaa gacatactgt catataatat 180
tattatacaa aagtagtaat tagtaatcgt gttacgtaat ataatgtacc agcccgggcc 240
gtcgaccacg cgtgccctat agtcactaac actcattaca ttcgtacaaa ctcattacac 300
tgtccattcc gttcctttct ggtttcgtta aatataaata aagcaaaaaa caccgtgatt 360
ccatgaacta aaaacacaag accccaatcc tgcaaccata acagcgtaaa aaaatctaca 420
gagctttagg aagagagaaa attaccgtca aaatgttggt ggcgttgtac tctaggacat 480
ctaattcagg ctgaataata acatccaatt caggtctatt atagaaacca gctttctgta 540
gattataatc ctgtgtgatt accgtcccag ataaagagat acgcgctccc attataagaa 600
gtgctctaca acatgtttct cgcacctttt catcaaaaag actagtgtca agagcagatg 660
taattgtatc aacagcttcc tctctatata tactgnactt ctgttgatcc acctgtcagt 720
tggaatacaa acatcttaac aatcaaatat gtgtgacaat ttt 763

<210> 14

<211> 21

<212> DNA

<213> primer

<400> 14
ttttactctt tgttataata g 21

<210> 15

<211> 20

<212> DNA

<213> primer

<400> 15

tttttagttc atggaatcac                                                20

<210>  16

<211>  22

<212>  DNA

<213>  primer

<400>  16
acactaacac tcattacatt cg                                              22

<210>  17

<211>  20

<212>  DNA

<213>  primer

<400>  17
cctaaagctc tgtagatttt                                                 20

<210>  18

<211>  22

<212>  DNA

<213>  primer

<400>  18
aaagcaaaaa acaccgtgat tc                                              22

<210>  19

<211>  21

<212>  DNA

<213>  primer

<400>  19
ggtgttatta ttcagcctga a                                               21